# EUROPEAN PATENT SPECIFICATION

(11) **EP 3 743 511 B1**
(45) Date of publication and mention of the grant of the patent: **07.06.2023**
(21) Application number: 19702676.8
(22) Date of filing: 23.01.2019
(51) Int. Cl.: C12N 5/0783, A61K 35/17

(54) **BIOMARKER PREDICTIVE OF TUMOUR INFILTRATING LYMPHOCYTE THERAPY AND USES THEREOF**
FÜR TUMORINFILTRIERENDE LYMPHOZYTENTHERAPIE PRÄDIKTIVER BIOMARKER UND SEINE VERWENDUNG
BIOMARQUEUR PRÉDICTIF DE LA THÉRAPIE DES LYMPHOCYTES INFILTRANT LES TUMEURS ET SES UTILISATIONS

(30) Priority: 23.01.2018 GB 201801067
(43) Date of publication of application: 02.12.2020
(73) Proprietor: INSTIL BIO (UK) LIMITED, Manchester M13 9XX (GB)
(72) Inventor: PRICE, Nicola Kaye, Wilmslow, Cheshire SK9 5DG (GB); BRIDGEMAN, John Stephen, Wilmslow, Cheshire SK9 5DG (GB)
(74) Representative: Murgitroyd & Company
(86) International application number: PCT/GB2019/050188
(87) International publication number: WO 2019/145711

(56) References cited:
- WO-A1-2017/179015
- STEFAN MEHRLE ET AL: "Enhancement of anti-tumor activity in vitro and in vivo by CD150 and SAP", MOLECULAR IMMUNOLOGY., vol. 45, no. 3, 1 February 2008 (2008-02-01), pages 796-804, XP055573683, GB ISSN: 0161-5890, DOI: 10.1016/j.molimm.2007.06.361
- MEGHEN B. BROWNING ET AL: "The T cell activation marker CD150 can be used to identify alloantigen-activated CD4+25+ regulatory T cells", CELLULAR IMMUNOLOGY., vol. 227, no. 2, 1 February 2004 (2004-02-01), pages 129-139, XP055573692, US ISSN: 0008-8749, DOI: 10.1016/j.cellimm.2004.03.003

## Description

### FIELD OF THE INVENTION

The present invention relates to the field of prognosis of cancer following treatment with T-cells including Tumour Infiltrating Lymphocytes (TILs). The prognosis is based on the quantification of a biological marker expressed by the T-cells including Tumour Infiltrating Lymphocytes. The invention also relates to the field of exploitation and/or manipulation of the biological markers to enhance the therapeutic efficacy of T-cell therapies including TIL therapy.

### BACKGROUND TO THE INVENTION

Adoptive cell therapy (ACT) using autologous T-cells to mediate cancer regression has shown much promise in early clinical trials. Several general approaches have been taken such as the use of naturally occurring tumour reactive or tumour infiltrating lymphocytes/Tumour associated lymphocytes (TILs) expanded ex vivo. Additionally, T-cells may be modified genetically to retarget them towards defined tumour antigens. This can be achieved via the gene transfer of: peptide (p)- major histocompatibility complex (MHC) specific T-cell Receptors (TCRs); or synthetic fusions between tumour specific single chain antibody fragment (scFv) and T-cell signalling domains (e.g. CD3ζ), the latter being termed chimeric antigen receptors (CARs). TIL and TCR transfer has proven particularly effective when targeting Melanoma (Rosenberg et al., 2011; Morgan et al., 2006), whereas CAR therapy has shown much promise in the treatment of certain B-cell malignancies (Grupp et al., 2013).

Tumour Infiltrating Lymphocyte therapy has been applied to a number of different malignancies including gastric (Xu et al., 1995), renal (Figlin et al., 1997; Goedegebuure et al., 1995), cervical (Stevanovic et al., 2015) and colorectal cancers (Gardini et al., 2004), but has been most widely applied and shown most development and promise in melanoma therapy. Trials in advanced metastatic melanoma have consistently shown around 50% response rate with 15-20% complete response (cure) (Rosenberg et al., 2011; Dudley et al., 2010). Additionally, Tumour associated lymphocytes can be obtained from ascites and grown in the same manner as TIL.

The process of TIL production offers lower costs and improved technological innovations compared to gene-modified T-cells; however, the process is more laborious and requires a higher degree of user skill to optimise growth. In current methodologies, tumour biopsies are taken from the patient and transferred to a laboratory setting. There are two options for TIL production: i) the tumour is cut into small fragments approx. 1-2mm³ and seeded in individual wells of 24-well tissue culture plates; or ii) the tumour is enzymatically digested and the resulting single cell suspension is cultured in 24-well tissue culture plates. In both cases the TIL are then cultured for 2-3 weeks with >3000 IU/ml IL-2, after which they undergo a two week expansion with irradiated feeder cells to obtain generally >1×10¹⁰ cells for infusion. During the expansion phase, the patient receives preconditioning chemotherapy (typically cyclophosphamide and fludarabine), and upon infusion of the cells receives supportive IL-2 treatment to enhance TIL engraftment.

With any therapeutic intervention there is a mixed patient response and as part of the refinement of the treatment the goal is then to determine which patients will show a clear benefit from the treatment given. Immunotherapeutics are no exception and as classical examples the pre-treatment mean corpuscular hemoglobin concentration has been shown to predict the outcome of TroVax vaccination (Harrop et al., 2012) and PDL1 expression has been used to define the patients who show more benefit from treatment with anti-PD1 therapy (Topalian et al. 2012). As TIL therapy in melanoma has around a 50% response rate, it would be beneficial to find markers which may predict those patients who will benefit most from treatment, particularly as agents used alongside the TIL are potentially very toxic (IL-2 and preconditioning chemotherapy). To this end it has been suggested that TIL products enriched in effector memory T-cells demonstrate better patient responses (Radvanyi et al., 2015). Furthermore, BTLA expression in TIL has been correlated with a good prognosis following TIL infusion (Radvanyi et al., 2012; Haymaker et al., 2015).

The disclosure provided herein relates to a cell surface marker which correlates with successful treatment following infusion and thus could be used as a marker of prognosis as TIL populations expressing this marker appear to show increased tumour reactivity as they are associated with improved clinical response rates.

The disclosure also describes how this receptor can be exploited to improve TIL and other T-cell therapies including therapies that utilise gene-modified T-cells.

### FIGURES

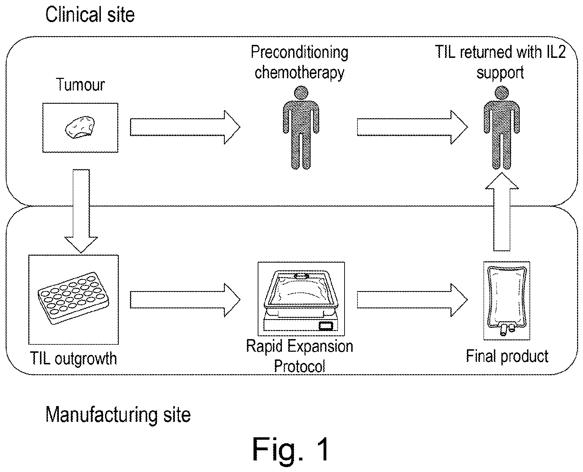

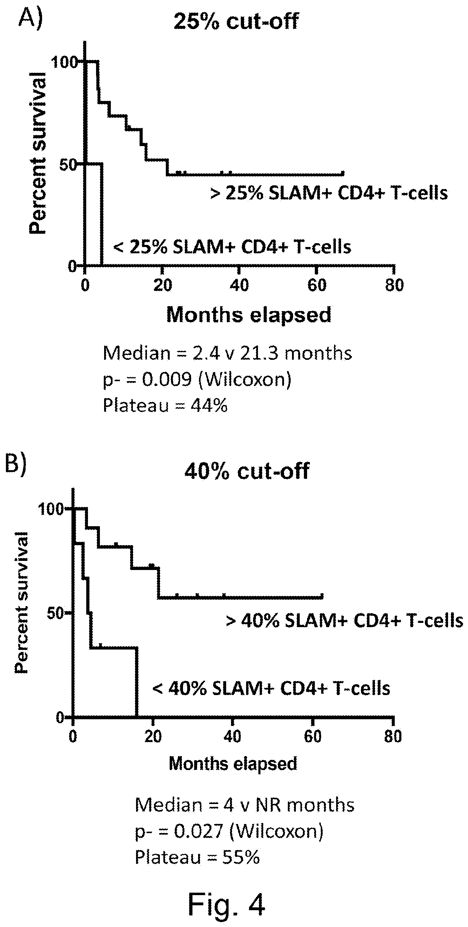

**Figure 1** - **TIL manufacturing process.** Current TIL therapy is critically dependent on two distinct sites. In the clinical site the tumour is resected and sent to the second site (the manufacturing site) where the tumour is dissociated and the T-cells grown out using IL-2 in plates. After approximately two weeks the cells are put into a rapid expansion protocol (REP) to grow the cells to >1×10¹⁰ in number. During the REP the patient undergoes preconditioning chemotherapy. Post-REP The TIL final product is returned to the patient along with intravenous IL-2 to support the reinfused T-cells.

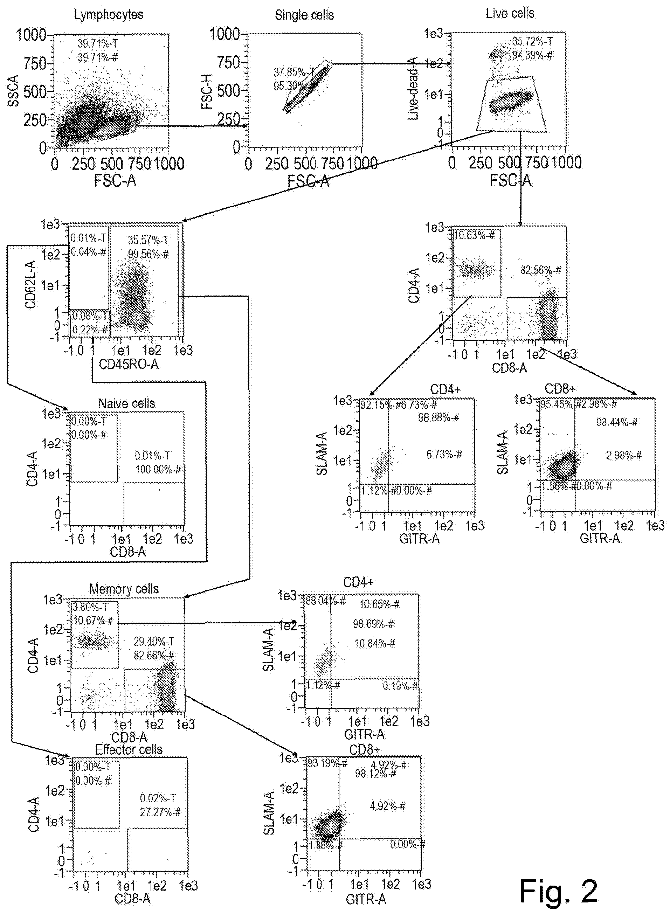

**Figure 2** - **Exemplar flow cytometric staining gating strategy for SLAM measurement.** Pre or Post-REP TIL were stained with the following antibodies:
   Fixable Viability Dye eFluor 450, αCD45RO FITC, αCD8 PE Vio770, αCD4 APC Cy7, αCD62L APC; and then counterstained with either pairs of mlgG1 PE and αSLAM PE. Cells were acquired on a MACSQuant analyser. Analysis was performed using MACSQuantify software using the gating strategies shown.
**Figure 3a** **- Expression of SLAM/CD150 in TIL pre- and post- rapid expansion protocol (REP).** Post-REP TIL were stained with the following:
   Fixable Viability Dye eFluor 450, αCD45RO FITC, αCD8 PE Vio770, αCD4 APC Cy7, αCD62L APC; and then counterstained with either mlgG1 PE or αSLAM PE. Cells were acquired on a MACSQuant analyser. Analysis was performed using MACSQuantify software. The proportion of SLAM⁺ cells were determined in each CD4+ or CD8+ population and plotted using Graphpad software. For all three graphs, patients are stratified by clinical response (progressive disease, stable disease and responders), Response rates indicated are the best response achieved by the patient as determined by response evaluation criteria in solid tumours (RECISTv 1.1) measurements (refer to Schwartz et al. Eur J Cancer 2016) * P < 0.05 . For all three graphs shown the mean +/- SEM is plotted for each cell and patient subtype. Significance determined using 2-way ANOVA, followed by Sidak's multiple comparison test. For the top graph (Final product (CD4+)), Memory: PD vs R, **p = 0.009. For the bottom graph (Final Product), CD4: PD vs SD, *p = 0.037; PD vs R, **p = 0.004; CD8: PD vs R, **p = 0.008.

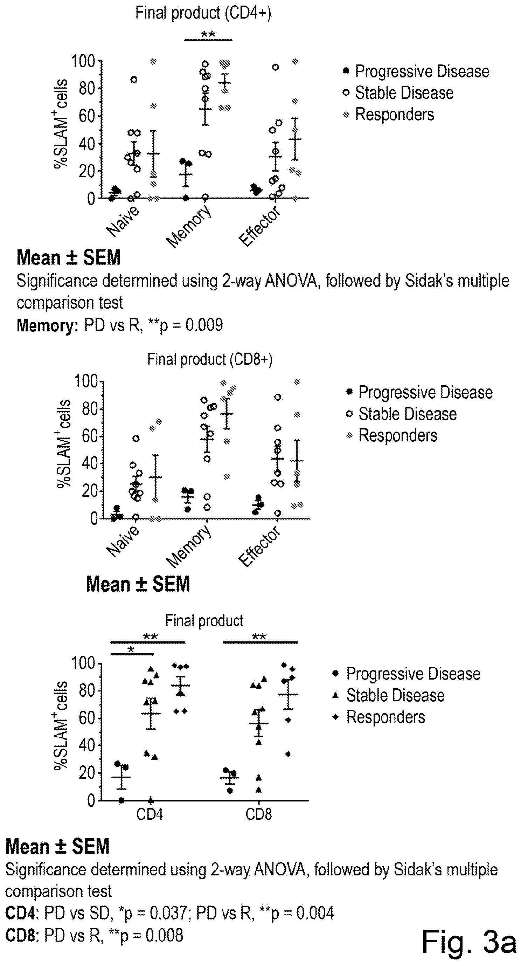

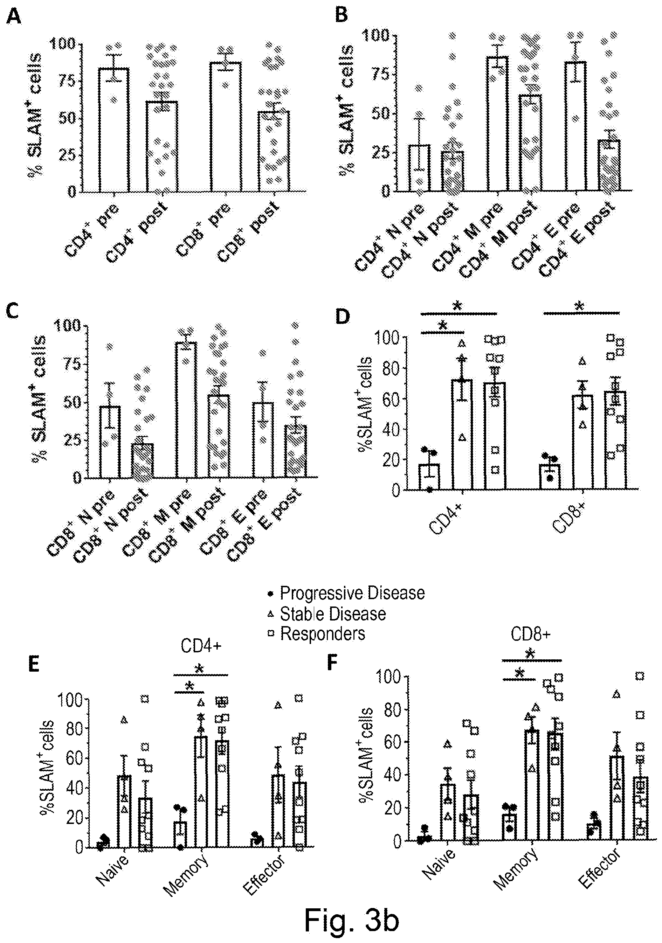

**Figure 3b** **- Expression of SLAM/CD150 in TIL pre- and post- rapid expansion protocol (REP).** Post-REP TIL were stained with the following:
   Fixable Viability Dye eFluor 450, αCD45RO FITC, αCD8 PE Vio770, αCD4 APC Cy7, αCD62L APC; and then counterstained with pairs of either mlgG1 PE and mlgG1 eFluor 710 isotype controls or αSLAM PE and αGITR eFluor 710. Cells were acquired on a MACSQuant analyser. Analysis was performed using MACSQuantify software. The proportion of SLAM+ cells were determined in each CD4+ or CD8+ population and plotted using Graphpad software. (**A - C**) SLAM expression in Pre-REP and Post-REP TIL from all melanoma subtypes; **A**) SLAM expression in all CD4+ and CD8+ T-cells; **B**) SLAM expression in naive [N], memory [M] and effector [E] CD4+ TIL; C) SLAM expression in naive [N], memory [M] and effector [E] CD8+ TIL; (**D - F**) SLAM expression in CD4+ and CD8+ TIL from cutaneous melanoma patients stratified by clinical response; **D**) SLAM expression on CD4+ and CD8+ TIL; E) SLAM expression in CD4+ T-cell subsets and **F**) SLAM expression in CD8+ subsets. Closed circles = progressive disease, open triangles = stable disease, open squares = responders. Response rates indicated are the best response achieved by the patient as determined by response evaluation criteria in solid tumours (RECISTv 1.1) measurements (refer to Schwartz et al. Eur J Cancer 2016) * P < 0.05
**Figure 4** **- Kaplan-Meier survival curve of patients correlating to SLAM expression -** overall survival times of patients is plotted with two groups: in a first graph (**A**) SLAM high treated (patients with greater than 25% SLAM positive CD4 T-cells) and SLAM low treated (patients with less than 25% SLAM positive T-cells); and in a second graph (**B**) SLAM high treated (patients with greater than 40% SLAM positive CD4 T-cells) and SLAM low treated (patients with less than 40% SLAM positive T-cells).

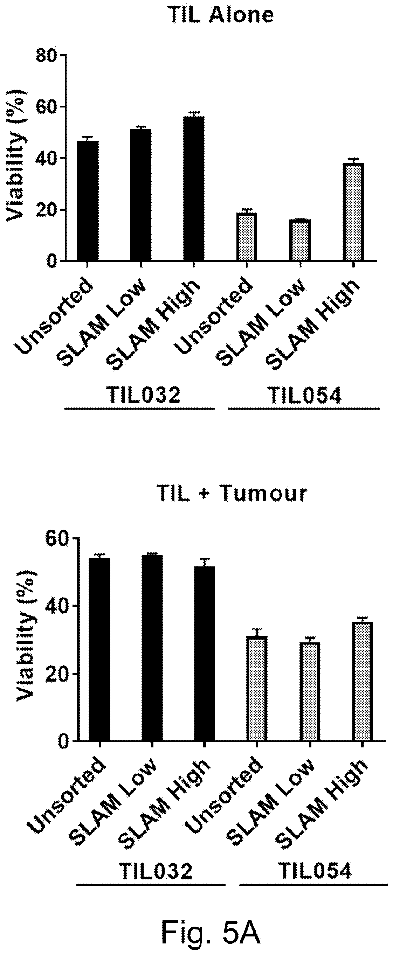

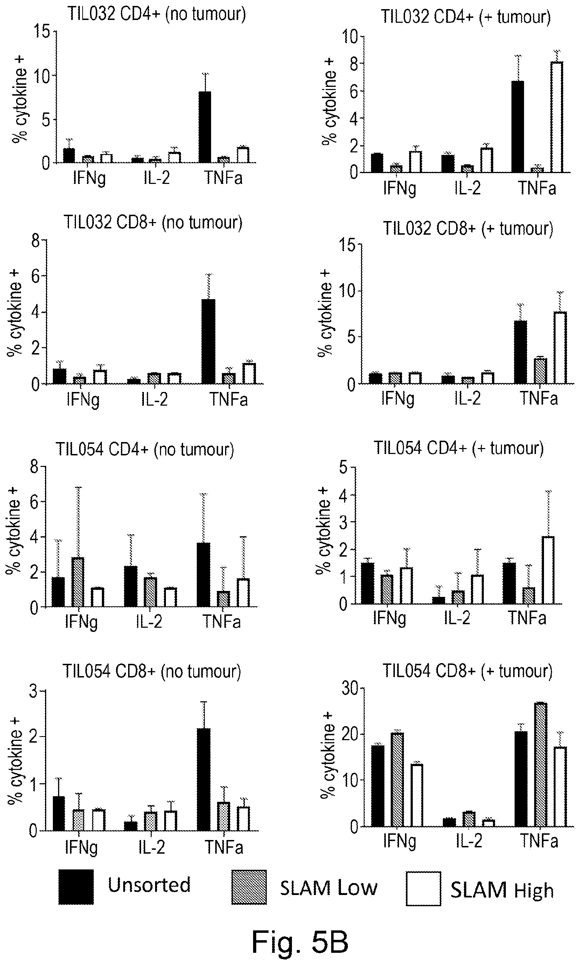

**Figure 5** - **Viability and cytokine response in SLAM sorted cells** - TIL from two donor final products, TIL032 and TIL054, were flow sorted for a SLAM High and SLAM low population. 24 h after culture viability was assessed (**A**) and the cells mixed with their respective matched autologous tumour cell line and cytokine response measured using flow cytometry (**B**).

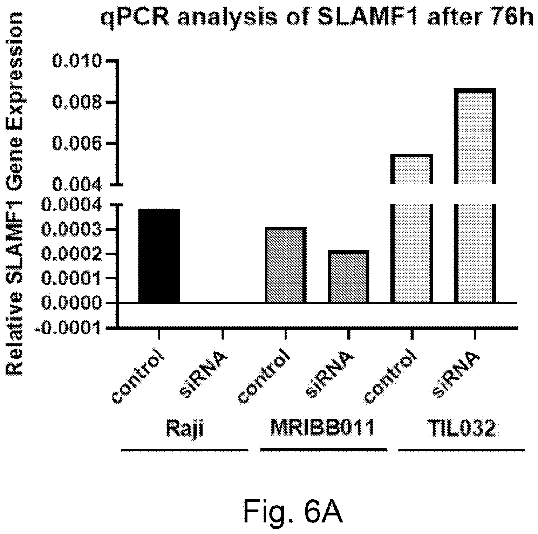

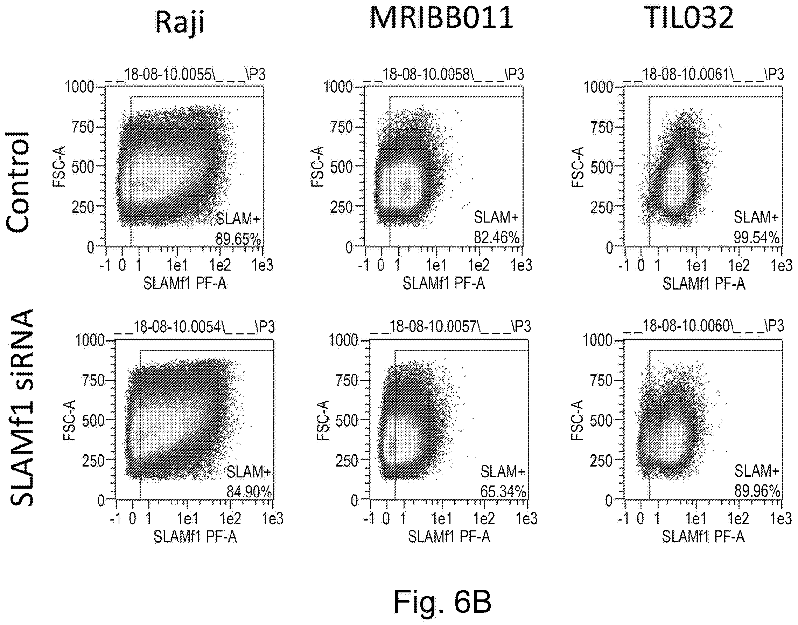

**Figure 6** - **SLAM siRNA** - SLAMF1 was measured by qPCR (**A**) or flow cytometry (**B**) in Raji, colorectal TIL (MRIBB011) and melanoma TIL (TIL032) following 76 h treatment with SLAMF1 siRNA (siRNA) or untreated cells (control).

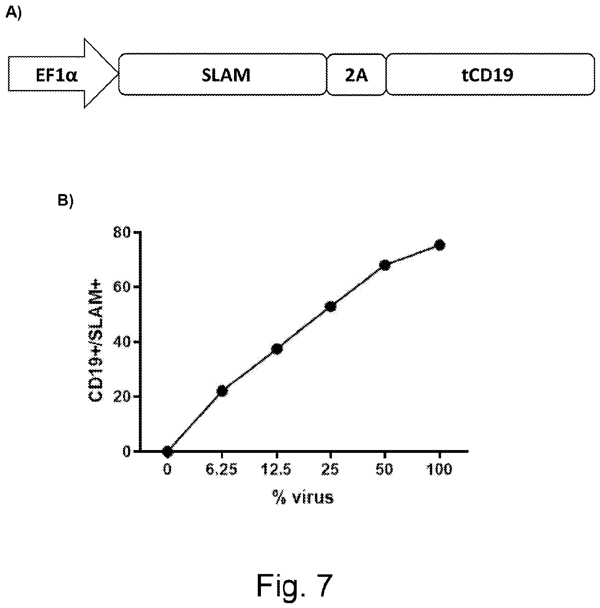

**Figure 7** - **SLAM overexpression** - A SLAM expression cassette was created by cloning the human SLAMF1 sequence, 2A cleavage sequence and human cytoplasmic domain truncated CD19 sequence downstream of an EF1α promoter (**A**). Jurkat JRT3-T3.5 cells were transduced with titrating concentrations of lentiviral particles containing the SLAMF1 and truncated CD19 genes and expression analysed by flow cytometry (**B**).

### SUMMARY OF ASPECTS OF THE INVENTION AND DISCLOSURE

The present inventors have identified a cell surface marker: Signaling lymphocytic activation molecule (SLAM/SLAMF1/SLAM family member 1/CD150/CDw150/IPO-3; Human amino acid sequence, see: NCBI Reference Sequence: NP_003028.1), which correlates with successful treatment following T-cell infusion (TIL infusion), thus the marker indicates that the T-cells (TILs) are likely to be tumour reactive. At present it is not known by what mechanism this increased tumour reactivity occurs, for example, it may be via enhanced tumour cell killing in the SLAM expressing population and/or, for example, SLAM expression could improve cell survival and persistence in the tumour microenvironment. The present disclosure relates to how this receptor can be exploited to improve TIL therapy and other cancer therapies that use T-cells, including therapies that utilise gene modified T-cells.

The references to the methods of treatment by therapy of this description are to be interpreted as references to compounds, pharmaceutical compositions and medicaments of the present invention for use in those methods.

In a first aspect of the invention, provided herein is a method for obtaining a cell population enriched for tumour reactive T-cells wherein T-cells expressing CD150/SLAM/SLAMF1 are selected and optionally expanded wherein the T-cells expressing CD150 are T-cells selected from cells originating from a patient and the cell population is selected from a population of tumour infiltrating lymphocytes (TILs) from a tumour biopsy, lymph node or ascites.. Thus cells expressing CD150/SLAM/SLAMF1 may be selected from a bulk population of cells, such as a T-cell population (e.g. gene modified T-cells) or a T cell population which includes a small proportion of other cell types (e.g. a TIL population).

The T-cells expressing CD150/SLAM/SLAMF1 are selected from cells originating from a patient (e.g. TIL cells from a tumour biopsy, lymph nodes, ascites). In embodiments, the selection of T-cells expressing CD150/SLAM/SLAMF1 comprises one or more of (i) flow cytometry, (ii) antibody panning, (iii) magnetic selection, (iv) biomarker targeted cell enrichment. The selection may comprise contacting the cell population with an anti- CD150/SLAM/SLAMF1 antibody. The selected cells may then be separated and expanded to enrich the number of CD150/SLAM/SLAMF1 positive (+ve) cells present in the population.

In embodiments, provided herein TILs expressing the biological marker CD150/SLAM/SLAMF1 are expanded by way of one or both of the following options:
a. Expansion with irradiated feeder cells to provide a T-cell activation signal and costimulation driven by antibodies or costimulatory receptors; and/or
b. Expansion with immobilised or soluble reagents which provide a T-cell activation signal and costimulation signals driven through said one or more biological markers.

The cell population according to the method of the invention is selected from: i) a population of tumour infiltrating lymphocytes (TILs) from a tumour biopsy, lymph node or ascites. Also disclosed is a cell population selected from a population of gene modified T-cells for example T-cells engineered to express a CAR and/or a TCR and/or other exogenous nucleic acid.

Also disclosed is a population of cells enriched for tumour reactive T-cells which has been obtained according to any of the methods provided herein. Such a population may have been obtained by starting with a TIL population or a population gene modified T-cells and selected and optionally enriching for CD150/SLAM/SLAMF1 positive (+ve) cells in that population, for example using any of the methods provided herein.

Also disclosed herein is a population of cells enriched for tumour reactive T-cells (such as TILs or gene modified T-cells), wherein >25% of the T-cells or TILs express biological marker CD150/SLAM/SLAMF1, or wherein >30%, >35% or >40% of the T-cells or TILs express biological marker CD150/SLAM/SLAMF1.

Also disclosed herein is a population of TILs enriched for biological marker CD150/SLAM/SLAMF1. Also disclosed is that the population of TILs enriched for biological marker CD150/SLAM/SLAMF1 is obtained according to a method described herein. According to this disclosure, the TILs may originate from a melanoma.

T cells (including T-cells obtained from a TIL population) according to this disclosure may be engineered to express or over-express CD150/SLAM/SLAMF1 from an exogenous nucleic acid. Expressing or over-expressing CD150/SLAM/SLAMF1 in such a manner may increase the tumour reactivity of the engineered T cell. In one aspect of the disclosure, provided herein is a T-cell comprising a first exogenous nucleic acid encoding CD150/SLAM/SLAMF1. The T--cell may further comprise a second exogenous nucleic acid encoding a Chimeric Antigen Receptor (CAR) and/or T-cell receptor (TCR) and/or other protein.

Suitably, the T-cell is a CD4+ or CD8+ cell. In some aspect of the disclosure, the T--cell is a memory cell.

Also disclosed is is a method of treating a disease in a subject comprising administering T-cells, such as TIL or gene modified T-cells, that express CD150/SLAM/SLAMF1 to the subject. The TILs may be a population of TILs enriched for CD150/SLAM/SLAMF1 by selection and expansion of cells originating from a subject or the TILs may be TILs or other T-cells that have been engineered to express CD150/SLAM/SLAMF1. The subject is typically a human. The method of treating is suitably adoptive cell therapy; T-cells may be autologous or allogenic. The disease is suitably cancer, e.g. melanoma, or ovarian or cervical cancer.

According to the invention, there is provided a population of tumour infiltrating lymphocytes (TILs) obtained from a tumour biopsy, lymph node or ascites of a patient and enriched for tumour reactive autologous T-cells expressing CD150 for use in the treatment of cancer adoptive cell therapy; T-cells may be autologous or allogenic. The disease is suitably cancer, e.g. melanoma, or ovarian or cervical cancer.

According to the invention, there is provided a population of tumour infiltrating lymphocytes (TILs) obtained from a tumour biopsy, lymph node or ascites of a patient and enriched for tumour reactive T-cells autologous to the patient expressing CD150 for use in the treatment of cancer in said patient wherein at least 25% of the T-cells express CD150.

Also disclosed herein are pharmaceutical compositions suitable for intravenous infusion comprising a population of cells enriched for tumour reactive T-cells expressing CD150/SLAM/SLAMF1 including T-cells engineered to express CD150/SLAM/SLAMF1 together with a pharmaceutically acceptable carrier, diluent or excipient, and optionally one or more further pharmaceutically active polypeptides and/or compounds.

Also provided herein according to the invention is a method for assessing the tumour reactivity of a cell population which method comprises quantifying T-cells in the cell population that are expressing SLAM/SLAMF1/CD150 wherein the cell population may be i) TILs from a patient and the T-cells expressing SLAM/SLAMF1/CD150 are quantified pre-REP and/or post-REP; or ii) a population of T-cells engineered to express an exogenous CAR and/or a TCR. When assessing SLAM/SLAMF1/CD150 expression levels, at least 25% of T-cells expressing SLAM/SLAMF1/CD150 in a population of T-cells/TILs indicates that the cell population is tumour reactive wherein said method is not a method of treatment or a diagnostic method performed on the animal or human body.

The disclosure described herein relates to an in vitro method for the prognosis of patients who may receive Tumour Infiltrating Lymphocyte (TIL) therapy for cancer and exploitation of these prognostic markers to develop novel methods to generate a more optimal TIL product; the method comprises the following:
i) Quantifying in a sample of tumour digest or TIL product during manufacture, from said patient, at least one biological marker (SLAM/CD150) on the TIL wherein the quantification is the proportion of cells expressing said biological marker (SLAM/CD150).
ii) Comparing the value obtained at step i) for said at least one biological marker with a predetermined reference value for the same biological marker; which predetermined reference value is correlated with a specific prognosis of progression of said cancer.
iii) Isolating cells from bulk TIL populations prior to, during or after manufacture based on enrichment of cells expressing the said biomarker(s).

According to this disclosure, the method, step i) consists of quantifying one or more biological markers by flow cytometry

According to this disclosure, the method, step i) consists of quantifying said biological marker by gene expression analysis in the whole tumour tissue sample.

According to this disclosure, the method, step i) consists of quantification of the said biological marker by immunohistochemistry of the entire tumour tissue sample.

According to this disclosure, the method, step iii) consists of isolating cells expressing the biomarker(s) using flow cytometric sorting

According to this disclosure, the method, step iii) consists of isolating cells expressing the biomarker(s) using some other form of physical separation technique which may include but is not limited to: Miltenyi MACS separation, StemCell Technologies magnetic separation technology or flow cytometric sorting.

According to this disclosure, the method, step iii) consists of enriching cells expressing the biomarker(s) via some form of process of mitogenic stimulation such as by using antibodies or soluble receptor protein which engages this Biomarker in combination with stimulation of T-cell activation such as to induce costimulation though the biomarker.

### DETAILED DESCRIPTION

### CELLS

The present disclosure relates to T-cells, for example T-cells present in a sample of Tumour-infiltrating lymphocytes (TIL). In particular, it relates to cell populations comprising T-cells such as populations of TILs which are enriched for tumour-reactive T-cells.

Tumour-infiltrating lymphocytes are white blood cells that have left the bloodstream and migrated into a tumour. They are mononuclear immune cells, a mix of different types of cells (i.e., T-cells, B-cells, NK cells, macrophages) in variable proportions, T-cells being by far the most abundant cells. They can often be found in the stroma and within the tumour itself.

TILs may specifically recognize, lyse, and/or kill tumour cells. The presence of lymphocytes in tumours is often associated with better clinical outcomes.

T-cells or T-lymphocytes are a type of lymphocyte that has a central role in cell-mediated immunity. They can be distinguished from other lymphocytes, such as B-cells and natural killer cells (NK cells), by the presence of a T-cell receptor (TCR) on the cell surface. There are various types of T-cell, as summarised below.

Cytolytic T-cells (TC cells, or CTLs) destroy virally infected cells and tumour cells, and are also implicated in transplant rejection. CTLs express the CD8 molecule at their surface. These cells recognize their targets by binding to antigen associated with MHC class I, which is present on the surface of all nucleated cells. Through IL-10, adenosine and other molecules secreted by regulatory T-cells, the CD8+ cells can be inactivated to an anergic state, which prevents autoimmune diseases such as experimental autoimmune encephalomyelitis.

Memory T-cells are a subset of antigen-specific T-cells that persist long-term after an infection has resolved. They quickly expand to large numbers of effector T-cells upon re-exposure to their cognate antigen, thus providing the immune system with "memory" against past infections. Memory T-cells comprise three subtypes: central memory T-cells (TCM cells) and two types of effector memory T-cells (TEM cells and TEMRA cells). Memory cells may be either CD4+ or CD8+. Memory T-cells typically express the cell surface protein CD45RO.

Regulatory T-cells (Treg cells), formerly known as suppressor T-cells, are crucial for the maintenance of immunological tolerance. Their major role is to shut down T-cell-mediated immunity toward the end of an immune reaction and to suppress auto-reactive T-cells that escaped the process of negative selection in the thymus.

Two major classes of CD4+ Treg cells have been described - naturally occurring Treg cells and adaptive Treg cells.

Naturally occurring Treg cells (also known as CD4+CD25+FoxP3+ Treg cells) arise in the thymus and have been linked to interactions between developing T-cells with both myeloid (CD11c+) and plasmacytoid (CD123+) dendritic cells that have been activated with TSLP. Naturally occurring Treg cells can be distinguished from other T-cells by the presence of an intracellular molecule called FoxP3.

Adaptive Treg cells (also known as Tr1 cells or Th3 cells) may originate during a normal immune response.

Natural Killer Cells (or NK cells) are a type of cytolytic cell which form part of the innate immune system. NK cells provide rapid responses to innate signals from virally infected cells in an MHC independent manner.

NK cells (belonging to the group of innate lymphoid cells) are defined as large granular lymphocytes (LGL) and constitute the third kind of cells differentiated from the common lymphoid progenitor generating B- and T-lymphocytes.

### SELECTION AND ENRICHMENT

In one aspect, the disclosure provided herein relates to obtaining and using populations of T-cells, including T-cells present in tumour-infiltrating lymphocytes (TILs) populations, that are enriched for tumour reactive T-cells by selecting T-cells expressing CD150/SLAM/SLAMF1 from the bulk population of cells. When selected, the CD150/SLAM/SLAMF1 positive cells are separated from the bulk population to provide a population enriched for T-cells expressing CD150/SLAM/SLAMF1, and optionally the selected cells are then expanded to increase the number of CD150/SLAM/SLAMF1 +ve (positive) cells. Once CD150/SLAM/SLAMF1 +ve cells have been selected for, any suitable T-cell expansion method known in the art can be used, providing CD150/SLAM/SLAMF1 expression is maintained post expansion.

For example, described herein is an in vitro method of selecting cells expressing prognostically-favourable levels of said biological marker (CD150/SLAM/SLAMF1) using one or more of the following selection techniques: (i) flow cytometry, (ii) antibody panning, (iii) magnetic selection, (iv) biomarker targeted cell enrichment.

Like many other human genes, there are multiple isoforms of SLAMF1, some of which are noncoding or do not generate a functional surface protein. However, these splice variants differ in their cytoplasmic domain, and antibodies to SLAMF1, such as those described herein, bind the extracellular domain and thus bind the different isoforms of SLAMF1.

Selected cells expressing the said biological marker (CD150/SLAM/SLAMF1) may then, for example, be expanded by way of one or both of the following options:
a) Irradiated feeder cells in such a fashion as to provide a T-cell activation signal and costimulation driven by antibodies or costimulatory receptors; and/or
b) Immobilised or soluble reagents which provide a T-cell activation signal and costimulation signals driven through said one or more biological markers.

For example, in some aspect of the disclosure costimulation through SLAM/CD150 using antibodies may be feasible as they have been shown to induce costimulation in the literature, see: Aversa G, Chang CC, Carballido JM, Cocks BG, de Vries JE. J Immunol. 1997 May 1;158(9):4036-44.

### EXOGENOUS NUCLEIC ACID MOLECULE

In one aspect, the present disclosure provides a cell which comprises an exogenous nucleic acid molecule encoding SLAM (Signaling lymphocyte activation molecule) /SLAMF1/CD150. The word "exogenous" means that the nucleic acid molecule is made by recombinant means and is introduced into the cell e.g. by way of a vector, such as a lentiviral vector. The cell is engineered to contain the nucleic acid molecule and to express (or over-express) SLAM/SLAMF1/CD150. Optionally the cell may further comprise a second exogenous nucleic acid for example encoding a Chimeric Antigen Receptor (CAR) or a T-cell receptor (TCR) and so also express a CAR or TCR.

As used herein, the terms "polynucleotide", "nucleotide", and "nucleic acid" are intended to be synonymous with each other.

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described herein to reflect the codon usage of any particular host organism in which the polypeptides are to be expressed, e.g. codon optimisation.

Nucleic acids according to the disclosure may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present disclosure, it is to be understood that the polynucleotides may be modified by any method available in the art. Such modifications may be carried out in order to enhance the in vivo activity or life span of the polynucleotides of interest.

### VECTORS

In an aspect, the present disclosure provides a vector which comprises a nucleic acid sequence or nucleic acid construct of the disclosure.

Such a vector may be used to introduce the nucleic acid sequence(s) or nucleic acid construct(s) into a host cell so that it expresses SLAM/SLAMF1/CD150.

The vector may, for example, be a plasmid or a viral vector, such as a retroviral vector or a lentiviral vector, or a transposon based vector or synthetic mRNA. Vectors derived from retroviruses, such as the lentivirus, are suitable tools to achieve long-term gene transfer since they allow long-term, stable integration of a transgene or transgenes and its propagation in daughter cells.

The vector may be capable of transfecting or transducing a T-lymphocyte. The present disclosure also provides vectors in which a nucleic acid of the present disclosure is inserted.

The expression of natural or synthetic nucleic acids encoding SLAM/SLAMF1/CD150, and optionally a TCR or CAR is typically achieved by operably linking a nucleic acid encoding the SLAM/SLAMF1/CD150 and TCR/CAR polypeptide or portions thereof to one or more promoters, and incorporating the construct into an expression vector. The vectors can be suitable for replication and integration in eukaryotic cells. Typical cloning vectors contain transcription and translation terminators, initiation sequences, and promoters useful for regulation of the expression of the desired nucleic acid sequence.

Viral vector technology is well known in the art and is described, for example, in Sambrook et al. (2001, Molecular Cloning: A Laboratory Manual, Cold Spring Harbor Laboratory, New York), and in other virology and molecular biology manuals, see also, WO 01/96584; WO 01/29058; and U.S. Pat. No.6,326,193).

In order to assess the expression of the SLAM/SLAMF1/CD150 polypeptide or portions thereof, the expression vector to be introduced into a cell can also contain either a selectable marker gene or a reporter gene or both to facilitate identification and selection of expressing cells from the population of cells sought to be transfected or transduced through viral vectors. For example, the marker gene may be CD19 as shown in Figure 7A.

According to the disclosure, the nucleic acid construct is as shown in Figure 7A. Figure 7A shows a lentiviral expression construct created in which SLAMF1 and a CD19 marker gene are driven by an EF1α promoter.

### PHARMACEUTICAL COMPOSITION

The present disclosure also relates to a pharmaceutical composition containing a population of cells, T-cells or TIL(s) of the disclosure together with a pharmaceutically acceptable carrier, diluent or excipient, and optionally one or more further pharmaceutically active polypeptides and/or compounds. Such a formulation may, for example, be in a form suitable for intravenous infusion. Provided by the disclosure herein is a pharmaceutical composition for intravenous infusion comprising a population of T-cells wherein at least 25%, 30%, 40%, or 50% of the T-cells express SLAM/SLAMF1/CD150.

### METHOD OF TREATMENT

A TIL product, for use in treatment, comprising TILs expressing SLAM/SLAMF1/CD150 may be obtained by enriching for cells expressing SLAM/SLAMF1/CD150 from cells originating from a subject or by engineering cells to express SLAM/SLAMF1/CD150.

T-cells including tumour infiltrating lymphocytes (TILs) expressing SLAM/SLAMF1/CD150 of the present disclosure may be ex vivo either from a patient's own peripheral blood (autologous), or in the setting of a haematopoietic stem cell transplant from donor peripheral blood (allogeneic), or peripheral blood from an unconnected donor (allogeneic). In these instances, T-cells expressing SLAM/SLAMF1/CD150 and, optionally, a CAR and/or TCR, are generated by introducing DNA or RNA coding for the S SLAM/SLAMF1/CD150 and, optionally, a CAR and/or TCR, by one of many means including transduction with a viral vector, transfection with DNA or RNA.

For purposes of the disclosed methods, wherein cells are administered to the patient, the cells can be T cells that are allogeneic or autologous to the patient.

A method for the treatment of disease relates to the therapeutic use of a vector or cell of the disclosure. In this respect, the vector, or T-cell may be administered to a subject having an existing disease or condition in order to lessen, reduce or improve at least one symptom associated with the disease and/or to slow down, reduce or block the progression of the disease. The method of the disclosure relates to increasing the tumour reactivity of T cells, such as T cells that are present in and make up the vast majority of a TIL cell population. Increased anti-tumour activity has been demonstrated by the data herein which shows that T-cells with increased SLAM/SLAMF1/CD150 correlates with improved clinical response in cancer patients treated with these cells. These cells may be considered tumour reactive T-cells. It is not known why increased SLAM expression correlates with improved clinical response (referred to herein as increased tumour reactivity), for example, it may be that SLAM is involved in increasing tumour killing or SLAM may be involved in promoting T-cell persistence.

Further details of aspects and aspects of the disclosure are below.

There now exists irrefutable evidence that the immune system can mount an effective response to many cancer types. This has led to the development of a number of cancer immunotherapies. These can be broadly classified into cell-based immunotherapies, and non-cell based. Non-cell-based immunotherapies have shown some exciting results. In particular the use of checkpoint blockade antibodies such as anti-PD1 (Nivolumab; Pembrolizumab), and PDL1 (Atezolizumab), or anti-CTLA4 (Ipilumumab) have had remarkable results in the clinical setting for advanced metastatic melanoma. Cell-based immunotherapies for cancer have had equally exciting results in early trials with CD19-CAR T-cells targeting B-cell malignancies in particular showing encouraging results.

TIL therapy is generally a simpler approach in that it requires no genetic modification and thus is appealing. In melanoma response rates of around 50% are generally observed. A trial in cervical cancer also showed 33% response rate. There are also a number of ongoing trials for other cancer indications including ovarian cancer.

Cancer response is measured using RECIST guidelines (version 1 published in 2000) and more recently updated in RESIST guidelines 1.1 (Eisenhauer et al 2009). Enabling the uniform assessment of the change in tumour burden, which is an important feature of the clinical evaluation of cancer therapeutics: both tumour shrinkage or no detectable disease i.e. the objective response (CR + PR), no change (stable disease (SD)) and disease progression (PD) which are useful endpoints in clinical trials to determine therapeutic efficacy and patient prognosis. The critical change in RESIST 1.1 was that it allows tumours to increase in size due to inflammation prior to shrinkage which is critical to allow therapeutics such as TIL to function where otherwise these therapies may be termed a failure.

The present disclosure provides a method for the prognosis of the outcome of treatment with Tumour Infiltrating Lymphocyte therapy in a patient, which novel method is based on the detection and/or the quantification of one or more biological markers, e.g. on the CD4+ and/or CD8+ Tumour Infiltrating Lymphocytes in the tumour, in the product during the TIL manufacturing process or in the TIL product prior to infusion.

By flow cytometric analysis of the TIL product the applicants have found a cell molecular marker expressed on the CD4+ and/or CD8+ T-cells within the TIL product which correlates with the response seen in the patient upon infusion.

The marker described herein which has been shown by the present inventors to correlate with patient response is SLAM (CD150).

It has been found according to the disclosure that there is a correlation between expression of the cell surface marker and the outcome of treatment with TIL.

The receptor identified is SLAM (Signaling lymphocyte activation molecule/SLAMF1 /CD150). SLAM is a name given to a particular member of the SLAM family of receptors, referring specifically to the SLAM family receptor 1 (SLAMF1). The SLAM family contains a number of other members including SLAMF3 (CD229), SLAMF4 (CD244) and SLAMF7 (CRACC/CD319). Most of these receptors are self-ligands, that is they bind to one another across haematopoietic cells. The cytoplasmic domain of SLAM family receptors contains immunoreceptor tyrosine-based switch motifs which associate with SAP (in T-cells) or EAT-2 (in NK-cells) protein adaptors. The role of SLAM family receptors remains somewhat unresolved. They can assist in immune responses in an activatory role or an inhibitory role, depending on the SLAM family receptor in question and in which cell it is expressed. CD150 itself has proven costimulatory function. SLAM engagement induces a TH1 phenotype of cytokines dominated by IFNy and it has thus been suggested that manipulation of SLAM may be beneficial for TH2 polarized disease (Quiroga et al 2004). Furthermore, it has been noted that SLAM is expressed to a higher degree in TH1 cells as compared to TH2 cells (Hamalainen et al 2000) which may partly explain the observation as to why it induces TH1 like cytokines. The final interesting point to make with regards CD150 is that it is the primary viral receptor for the measles virus (Erlenhoefer et al 2001). This has been exploited for cell therapy purposes by using lentiviruses which are pseudotyped with the measles virus envelope to more specifically target lentiviruses to T-cells (Frecha et al. 2011)

Thus, a first object of the present disclosure consists of an in vitro method for the prognosis of patients who may receive Tumour Infiltrating Lymphocyte (TIL) therapy for cancer; the method comprises the following:-
i) quantifying in a sample of tumour digest, TIL material during the manufacturing process or TIL product, from said patient, at least one biological marker on the TIL; and
ii) comparing the value obtained at step i) for said at least one biological marker with a predetermined reference value for the same biological marker; which predetermined reference value is correlated with a specific prognosis of progression of said cancer.

Although there are previous examples of molecular markers on TIL which appear to correlate with clinical efficacy (for example BTLA), none have described SLAM. Radvanyi et al. 2015 have described the proportion of CD8+ T-cells within TILs as being associated with good clinical outcome. This is confounded by the observation that BTLA can act as a negative regulator of T-cell activity (Watanabe et al. 2003)

TIL products enriched for CD8+ cells does not confer a survival advantage over mixed CD4+ and CD8+ cells (Dudley et al., 2010). This is largely assumed to be because the presence of CD4+ helper T-cells assists the CD8+ T-cell survival and engraftment. Furthermore, there is evidence that CD4+ cells can assist in direct recognition and killing of tumour cells (Tran et al., 2014).

Radvanyi et al. (2015) also described the expression of BTLA as having favorable association with clinical benefit. The expression of BTLA has been associated with less differentiated T-cells with enhanced survival properties (Haymaker et al., 2015). However, paradoxically it has also been shown that the presence of more differentiated effector memory T-cells within TIL infusion products is associated with favorable outcome. Effector memory T-cells are usually marked by the co-expression of CD62L or CCR7 combined with CD45RO or CD45RA such that effector memory cells can have the following phenotypes: CD62L-/CD45RO+, CD62L-/CD45RA-, CCR7-/CD45RO+ or CCR7-/CD45RA-.

There are also a number of publications and prior art works describing associations with various cell surface markers on TIL in vivo with clinical outcome, but this is not associated with TIL manufacture, rather the analysis of tumour biopsies. Examples include patent US20090215053A1 - Vitro Method for the Prognosis of Progression of a Cancer and of the Outcome in a Patient and Means for Performing Said Method

As intended herein, Tumour Infiltrating Lymphocytes are a) CD45+ Cells isolated directly from the tumour sample of a patient with cancer via physical or enzymatic disaggregation, b) CD45+ cells isolated from lymph nodes from said patient c) CD45+ cells isolated from ascites (otherwise termed tumour associated lymphocytes). The TIL remain as such throughout the TIL manufacturing process with a propensity for them to obtain a slightly different phenotype wherein they remain CD45+ but the proportion of CD3+ cells increases as the cells are cultured in IL-2 and activated with irradiated feeder cells or an alternative TIL expansion system. These CD45+/CD3+ cells are termed T-cells or T-lymphocytes. As such the term 'TIL' encompasses any CD45+ cell from the point of surgery to the point of infusion back to the same patient.

The analysis of said markers (SLAM/SLAMF1/CD150) may be performed by one of several mechanisms. In the first instance flow cytometry may be performed. In this instance the cells may be stained with antibodies to SLAM to determine its presence or absence. Furthermore other antibodies may be incorporated into the staining panel to look at defined populations of cells within the TIL. For example the cells may be counterstained with antibodies to CD62L, CD45RO, CD4 and/or CD8.

The said markers (SLAM/SLAMF1/CD150) may be otherwise exploited in such a fashion as to enrich cells expressing said markers in an effort to improve the efficacy of the final product. For example flow cytometry can be taken advantage of to isolate cells expressing said markers by using specific antibodies (anti-SLAM) or recombinant (r) protein (for example r-SLAM) conjugated to fluorophores or other direct or indirect selectable markers.

Alternatively, other technologies for isolating cells may be performed. For example Miltenyi MACS magnetic technology, Invitrogen Dynal technology or StemCell Technologies EasySep technology may be used to isolate cells expressing said markers.

Alternatively yet, antibodies (or antibody fragments thereof) or recombinant protein either immobilized to plates, beads or other solid matrix, or expressed on an artificial antigen presenting cell platform may be used to enrich cells expressing said markers. In such examples the antibodies or recombinant protein may be found alone or in combination with an antibody or other activation platform which induces a primary activation signal to T-cells (examples include but are not limited to: Phytohaemagglutinin, anti-CD3 antibody, Peptide-major histocompatability antigen complex, phorbol myristate acetate).

The goal of all of the above is then to stratify patients based on expression of said markers, and where possible to isolate and/or enrich the therapeutic cells via one or more methods described above.

As intended herein, multiple methods are available in the art to assess the presence of the biomarker either using: (a) direct methods where biomarker binding moieties are bound to secondary reporting moieties in a single or multiple step process such as biomarker binding antibodies conjugated to a reporter system that can be detected such as those commonly used in flow cytometry. Microscopy or proteomic gel chromatography; or (b) indirect methods where biomarker encoding nucleic acid are quantified such as quantitative PCR. Where the method of choice is analysing cells on a single cell and population based method wherein cells are loaded with antibodies to defined surface markers which may be directly or indirectly coupled to fluorophores, which emit light at defined wavelengths which can be detected by components of the flow cytometer machine. Herein the term flow cytometry is preferred to the more commonly used but incorrect term FACS which is a trademark of Becton Dickinson flow cytometry machines. As such any flow cytometer may be used for the analysis (Becton Dickinson [BD], Miltenyi, Acea etc) for the purposes of this method but other methods may be employed.

Preferably, when step a) consists of the expression analysis of one or more genes, i.e. one or more pertinent biological markers, then the quantification of the expression of the said one or more genes is performed from the whole tumor tissue sample.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Certain aspects and embodiments of the invention will now be illustrated by way of example and with reference to the figures described above and tables described below.

### Example 1 - Analysis of expression of SLAM/CD150 in tumour infiltrating lymphocytes

Metastatic melanoma tumour biopsies were taken from patients and brought to the lab where they were first cut into fine pieces using a scalpel, and then digested to a single cell suspension using a mixture of collagenase and DNase.

The cell suspension was seeded at approximately 1×10⁶ viable CD3+ cells per well of a 24-well plate in complete media with the addition of 3000 IU/ml IL-2. Cultures were monitored for cell growth and split accordingly as needed to maintain a healthy culture.

The TIL are grown for up to 21 days or until the CD3+ cell count was greater than 30×10⁶ after which the cells were washed and frozen. Prior to freezing a sample was taken for flow cytometric analysis. At this point the sample is referred to as Pre-REP. Before the cells are ready for infusion they need to be expanded to numbers in excess of 1×10¹⁰. To achieve this the TIL undergo a rapid expansion protocol (Dudley et al., 2003). In brief, the TIL cells were defrosted 1 to 3 days prior to expansion. The TIL are then mixed at a 1:50 to 1:1000 ratio with irradiated auto/allo-geneic PBMC feeder cells with the addition of 10 - 50 ng/ml OKT3. The TIL were then expanded for a period of 2 weeks after which they are referred to as Post-REP TIL. Prior to infusion of the Therapeutic TIL product the (i.e. Post-REP TIL) the patient underwent pre-conditioning chemotherapy consisting of Fludarabine and Cyclophasphamide. The Post-REP TIL are issued to the patient and given as a single infusion prior to a number of doses of IL-2.

At the point of Pre-REP and Post-REP the TIL cells were analysed by flow cytometry. In brief, three samples of 1-2×10⁵ cells were washed in PBS and then incubated with 50 µl of 1:400 dilution of Fixable Viability Dye eFluor 450 for five minutes at room temperature in the dark. Cells were washed twice with 150 µl of PBS and then resuspended in 50 µl cold PBS supplemented with 2mM EDTA and 0.5% foetal calf serum (PEF). To each sample antibodies were added as indicated in the table below for 30 minutes at 4°C.

| **Antibody/Dye** | **Supplier** | **Concentration** | **Sample** | | |
|---|---|---|---|---|---|
| | | | **1** | **2** | **3** |
| Fixable Viability Dye eFluor 450 | ebioscience | 1:400 | y | y | Y |
| CD45RO FITC | Miltenyi | 1:25 | y | y | Y |
| CD8 PE Vio770 | Miltenyi | 1:25 | y | y | Y |
| CD4 APC Cy7 | Biolegend | 1:25 | y | y | Y |
| CD62L APC | Biolegend | 1:25 | y | y | Y |
| mlgG1 PE | Biolegend | 1:25 | y | - | - |
| mlgG1 PerCP eFluor 710 | ebioscience | 1:25 | y | - | - |
| SLAM PE | Miltenyi | I 1:25 | - | y | - |
| GITR PerCP eFluor 710 | ebioscience | 1:25 | - | y | - |

After incubation the cells were washed twice with 150 µl cold PEF and finally resuspended in 200 µl PBS. Cells were acquired on a MACSQuant analyser and data analysed using MACSQuantify software (Figure 1). When comparing TIL at Pre-REP and Post-REP we found enhanced expression of SLAM in the CD4+ and CD8+ TIL at the Pre-REP stage compared to the Post-REP stage. In particular we observed two distinct populations of SLAM expression in the CD4+ Post-REP TIL suggesting a group of patients who were SLAM-high and SLAM-low. In particular the SLAM expression tended to be largely restricted to the memory cell populations in the CD4+ and CD8+ populations, with lower expression in the naive and effector populations. The naive and effector populations form a small portion of the overall TIL products.

When we analyzed the proportion of SLAM+ cells with respect to patient response in cutaneous melanoma we found a significantly increased proportion of CD4+ T-cells which were SLAM+ in the patients who had stable disease (p = 0.0252) or responders (p = 0.0113), compared to those who had progressive disease ('progressors'). In the CD8+ population the significant difference observed was between those patients who had a response to treatment and those who had progressive disease (p = 0.0248). The significant differences were mainly restricted to the differences seen in the memory cell populations. In CD4+ T-cells the memory cell population had a significant difference between the patients with stable disease and progressors (p = 0.0384) and between progressive disease and responders (p = 0.0221). In the CD8+ memory T-cells we saw a significant difference between patients with progressive disease and stable disease (p = 0.0348) and between those with progressive disease and responders (p = 0.0169).

Following treatment the patients were evaluated to measure the tumour size according to response evaluation criteria in solid tumours (RECISTv1.1) measurements. The best response was determined for each patient according to the latest RECIST 1.1 guidelines (Schwartz et al. 2016). We plotted overall survival of patients against duration of response (Figure 4). We used two cut-offs for SLAM-high and SLAM-low: 25% and 40% of all CD4+ TIL in the final product respectively. We found that patients who had >25 % SLAM+ cells had enhanced survival compared to those that had <25 % SLAM+ cells (Median 21.3 months v 2.4 months respectively, with a significant difference of p = 0.009 by Wilcoxon test, and a plateau of 44 % (Figure 4A). Equally we found that patients who had >40 % SLAM+ cells had enhanced survival compared to those that had <40 % SLAM+ cells (Median Not Reached v 4 months respectively, with a significant difference of p = 0.027 by Wilcoxon test, and a plateau of 55 % (Figure 4A).

### Example 2 - modulation of SLAM expression in T-cells and TIL

We first assessed the impact of SLAM expression in TIL on the viability and functional response of TIL towards matched tumour (TIL032 and TIL054). To this end we took two TIL infusion products and sorted a SLAM-high and -low population using anti-SLAM antibodies and a flow sorter. Following an overnight rest period the cells were co-cultured with their autologous tumour line, or left in wells unstimulated, for 16 h and then the viability determined using DRAQ7 (Figure 5A) and the proportion of cells producing IL-2, IFNy and TNFα was determined using flow cytometry (Figure 5B). We found that TIL032 and TIL054 SLAM-high cells had increased viability compared to SLAM-low or unsorted cells (Figure 5A). When mixed with their tumour the effect was less obvious, but apparent in TIL054. When we assessed cytokine production we found elevated cytokine responses when the TIL were cultured with their matched tumour. This was most obvious when looking at production of TNFα. However, we found that the TNFα response from the SLAM-high sorted populations were, on the whole, greater than from the SLAM-low sorted cells, with the exception of the CD8+ response to tumour in TIL054.

Next we assessed impact of SLAMF1 siRNA on expression of SLAM in cell lines and T-cells as an approach to modulate SLAM expression. We took the SLAM+ cell line Raji and two TIL samples (MRIBB011 a colorectal tumour TIL sample and the TIL032 infusion product from TIL032). The cells were plated in 96 wells (1×10⁵ per well) with 10 µM of self-delivery siRNA (Accell Human SLAMF1 siRNA SMARTpool, 5 nmol - [Dharmacon, Colorado, USA]) in minimal media (RPMI + 1% FCS + ITS, and for the TILs supplemented with 1000 lU/ml of IL-2). Controls were grown at the same conditions but without the siRNA. 76h later 2×10⁵ cells (2x wells) from each condition were pooled and the Cells-to-CT^{™} 1-Step TaqMan^{®} Kit (Invitrogen) was used.

Briefly the cells were spun down, washed with PBS (R/T) and pelleted again. Supernatant was removed and 49 µl Lysis solution + 1 µl DNAse was added. They were incubated for 5 min at R/T and the 5 µl of STOP solution was added. Samples were left for 2 min at RT and then placed on ice until used. All above reagents are provided with the kit. The lysates are stable for up to 5 months at -80.

TaqMan reactions for SLAMF1 (Assay ID: Hs00234149_m1, Invitrogen) and GAPDH (Assay ID: Hs03929097_g1, Invitrogen) were set in triplicate, and the reaction volume was scaled down to 10 µl from the 20 µl suggested by the kit. 1 µl of the lysis was added in each reaction. A positive control reaction with already extracted RNA from untreated Raji cells was also set up to account for possible PCR inhibiting agents present in the cellular lysates.

We observed an almost complete knock down of SLAM transcript in Raji cells (>99%) (Figure 6A). In MRIBB011 we saw a 31% reduction of SLAM transcript; and in TIL032 we saw a 57% increase in SLAM transcript. Protein expression was altered to a different degree (Figure 6B). In Raji cells surface expression of the protein over the same time course decreased by 5.3%, in MRIBB011 TIL by 30.8% and in TIL032 by 9.6%. Thus SLAM siRNA is a suitable means of modulating SLAM expression, although optimisation is required for each cell line tested to observe the optimal knock down in expression.

Next we determined whether enhancement of SLAM expression in T-cells could provide an alternative approach to quantifying the impact of SLAM expression, rather than by knock-down as achieved via siRNA. To this end a lentiviral expression construct was created in which SLAMF1 and a CD19 marker gene are driven by and EF1α promoter (Figure 7A). Lentiviral particles were generated in HEK293T cells by transient transfection and then the resulting particles titrated on SLAM-negative Jurkat JRT3-T3.5 cells (Figure 7B). Co-expression of SLAM and CD19 was demonstrated in the Jurkat cells.

### References

Aspeslagh S, Postel-Vinay S, Rusakiewicz S, Soria JC, Zitvogel L, Marabelle A. Eur J Cancer. 2016 Jan;52:50-66. Rationale for anti-OX40 cancer immunotherapy.

Dudley ME, Wunderlich JR, Shelton TE, Even J, Rosenberg SA. J Immunother. 2003 Jul-Aug;26(4):332-42. Generation of tumor-infiltrating lymphocyte cultures for use in adoptive transfer therapy for melanoma patients.

Dudley ME, Gross CA, Langhan MM, Garcia MR, Sherry RM, Yang JC, Phan GQ, Kammula US, Hughes MS, Citrin DE, Restifo NP, Wunderlich JR, Prieto PA, Hong JJ, Langan RC, Zlott DA, Morton KE, White DE, Laurencot CM, Rosenberg SA. Clin Cancer Res. 2010 Dec 15;16(24):6122-31. CD8+ enriched "young" tumor infiltrating lymphocytes can mediate regression of metastatic melanoma.

Eisenhauer EA, Therasse P, Bogaerts J, Schwartz LH, Sargent D, Ford R, Dancey J, Arbuck S, Gwyther S, Mooney M, Rubinstein L, Shankar L, Dodd L, Kaplan R, Lacombe D, Verweij J. Eur J Cancer. 2009 Jan;45(2):228-47 New response evaluation criteria in solid tumours: revised RECIST guideline (version 1.1).

Erlenhoefer C, Wurzer WJ, Löffler S, Schneider-Schaulies S, ter Meulen V, Schneider-Schaulies J. J Virol. 2001. 75(10):4499-505 CD150 (SLAM) is a receptor for measles virus but is not involved in viral contact-mediated proliferation inhibition. J Virol. 2001 May;

Figlin RA, Pierce WC, Kaboo R, Tso CL, Moldawer N, Gitlitz B, deKernion J, Belldegrun A. J Urol. 1997 Sep;158(3 Pt 1):740-5. Treatment of metastatic renal cell carcinoma with nephrectomy, interleukin-2 and cytokine-primed or CD8(+) selected tumor infiltrating lymphocytes from primary tumor.

Frecha C, Lévy C, Costa C, Nègre D, Amirache F, Buckland R, Russell SJ, Cosset FL, Verhoeyen E. J Virol. 2011 Jun;85(12):5975-85. Measles virus glycoprotein-pseudotyped lentiviral vector-mediated gene transfer into quiescent lymphocytes requires binding to both SLAM and CD46 entry receptors.

Gardini A, Ercolani G, Riccobon A, Ravaioli M, Ridolfi L, Flamini E, Ridolfi R, Grazi GL, Cavallari A, Amadori D.J Surg Oncol. 2004 Jul 15;87(1):46-52. Adjuvant, adoptive immunotherapy with tumor infiltrating lymphocytes plus interleukin-2 after radical hepatic resection for colorectal liver metastases: 5-year analysis.

Goedegebuure PS, Douville LM, Li H, Richmond GC, Schoof DD, Scavone M, Eberlein TJ. J Clin Oncol. 1995 Aug;13(8):1939-49. Adoptive immunotherapy with tumor-infiltrating lymphocytes and interleukin-2 in patients with metastatic malignant melanoma and renal cell carcinoma: a pilot study.

Grupp SA, Kalos M, Barrett D, Aplenc R, Porter DL, Rheingold SR, Teachey DT, Chew A, Hauck B, Wright JF, Milone MC, Levine BL, June CH. N Engl J Med. 2013 Apr 18;368(16):1509-18. Chimeric antigen receptor-modified T cells for acute lymphoid leukemia.

Hamalainen H, Meissner S, Lahesmaa R. J Immunol Methods. 2000 Aug 28;242(1-2):9-19. Signaling lymphocytic activation molecule (SLAM) is differentially expressed in human Th1 and Th2 cells.

Harrop R, Treasure P, de Belin J, Kelleher M, Bolton G, Naylor S, Shingler WH. Cancer Immunol Immunother. 2012 Dec;61(12):2283-94. Analysis of pre-treatment markers predictive of treatment benefit for the therapeutic cancer vaccine MVA-5T4 (TroVax).

Haymaker CL, Wu RC, Ritthipichai K, Bernatchez C, Forget MA, Chen JQ, Liu H, Wang E, Marincola F, Hwu P, Radvanyi LG. Oncoimmunology. 2015 Mar 16;4(8): BTLA marks a less-differentiated tumor-infiltrating lymphocyte subset in melanoma with enhanced survival properties.

Knee DA, Hewes B, Brogdon JL.Eur J Cancer. 2016 Nov;67:1-10. Rationale for anti-GITR cancer immunotherapy.

Kochenderfer JN, Dudley ME, Kassim SH, Somerville RP, Carpenter RO, Stetler-Stevenson M, Yang JC, Phan GQ, Hughes MS, Sherry RM, Raffeld M, Feldman S, Lu L, Li YF, Ngo LT, Goy A, Feldman T, Spaner DE, Wang ML, Chen CC, Kranick SM, Nath A, Nathan DA, Morton KE, Toomey MA, Rosenberg SA. J Clin Oncol. 2015 33(6):540-9. Chemotherapy-refractory diffuse large B-cell lymphoma and indolent B-cell malignancies can be effectively treated with autologous T cells expressing an anti-CD19 chimeric antigen receptor.

Morgan RA, Dudley ME, Wunderlich JR, Hughes MS, Yang JC, Sherry RM, Royal RE, Topalian SL, Kammula US, Restifo NP, Zheng Z, Nahvi A, de Vries CR, Rogers-Freezer LJ, Mavroukakis SA, Rosenberg SA. Science. 2006 Oct 6;314(5796):126-9. Cancer regression in patients after transfer of genetically engineered lymphocytes.

Petty JK, He K, Corless CL, Vetto JT, Weinberg AD. Am J Surg. 2002 May;183(5):512-8. Survival in human colorectal cancer correlates with expression of the T-cell costimulatory molecule OX-40 (CD134).

Quiroga MF, Martinez GJ, Pasquinelli V, Costas MA, Bracco MM, Malbrán A, Olivares LM, Sieling PA, Garcia VE. J Immunol. 2004 Sep 15;173(6):4120-9. Activation of signaling lymphocytic activation molecule triggers a signaling cascade that enhances Th1 responses in human intracellular infection.

Radvanyi LG, Bernatchez C, Zhang M, Fox PS, Miller P, Chacon J, Wu R, Lizee G, Mahoney S, Alvarado G, Glass M, Johnson VE, McMannis JD, Shpall E, Prieto V, Papadopoulos N, Kim K, Homsi J, Bedikian A, Hwu WJ, Patel S, Ross MI, Lee JE, Gershenwald JE, Lucci A, Royal R, Cormier JN, Davies MA, Mansaray R, Fulbright OJ, Toth C, Ramachandran R, Wardell S, Gonzalez A, Hwu P.Clin Cancer Res. 2012 Dec 15;18(24):6758-70. Specific lymphocyte subsets predict response to adoptive cell therapy using expanded autologous tumor-infiltrating lymphocytes in metastatic melanoma patients.

Rapoport AP, Stadtmauer EA, Binder-Scholl GK, Goloubeva O, Vogl DT, Lacey SF, Badros AZ, Garfall A, Weiss B, Finklestein J, Kulikovskaya I, Sinha SK, Kronsberg S, Gupta M, Bond S, Melchiori L, Brewer JE, Bennett AD, Gerry AB, Pumphrey NJ, Williams D, Tayton-Martin HK, Ribeiro L, Holdich T, Yanovich S, Hardy N, Yared J, Kerr N, Philip S, Westphal S, Siegel DL, Levine BL, Jakobsen BK, Kalos M, June CH. Nat Med. 2015 Aug;21(8):914-21. NY-ESO-1-specific TCR-engineered T cells mediate sustained antigen-specific antitumor effects in myeloma.

Rosenberg SA, Yang JC, Sherry RM, Kammula US, Hughes MS, Phan GQ, Citrin DE, Restifo NP, Robbins PF, Wunderlich JR, Morton KE, Laurencot CM, Steinberg SM, White DE, Dudley ME. Clin Cancer Res. 2011 Jul 1;17(13):4550-7. Durable complete responses in heavily pretreated patients with metastatic melanoma using T-cell transfer immunotherapy.

So T, Croft M. J Immunol. 2007. Cutting edge: OX40 inhibits TGF-beta- and antigen-driven conversion of naive CD4 T cells into CD25+Foxp3+ T cells.

Shimizu J, Yamazaki S, Takahashi T, Ishida Y, Sakaguchi S.

Nat Immunol. 2002 Feb;3(2):135-42. Stimulation of CD25(+)CD4(+) regulatory T cells through GITR breaks immunological self-tolerance.

Schwartz LH, Litière S, de Vries E, et al. RECIST 1.1-Update and clarification: From the RECIST committee. Eur J Cancer. 2016;62:132-7.

Stevanovié S, Draper LM, Langhan MM, Campbell TE, Kwong ML, Wunderlich JR, Dudley ME, Yang JC, Sherry RM, Kammula US, Restifo NP, Rosenberg SA, Hinrichs CS. J Clin Oncol. 2015 May 10;33(14):1543-50. Complete regression of metastatic cervical cancer after treatment with human papillomavirus-targeted tumor-infiltrating T cells.

Topalian SL, Hodi FS, Brahmer JR, Gettinger SN, Smith DC, McDermott DF, Powderly JD, Carvajal RD, Sosman JA, Atkins MB, Leming PD, Spigel DR, Antonia SJ, Horn L, Drake CG, Pardoll DM, Chen L, Sharfman WH, Anders RA, Taube JM, McMiller TL, Xu H, Korman AJ, Jure-Kunkel M, Agrawal S, McDonald D, Kollia GD, Gupta A, Wigginton JM, Sznol M. N Engl J Med. 2012 Jun 28;366(26):2443-54. Safety, activity, and immune correlates of anti-PD-1 antibody in cancer.

Tran E, Turcotte S, Gros A, Robbins PF, Lu YC, Dudley ME, Wunderlich JR, Somerville RP, Hogan K, Hinrichs CS, Parkhurst MR, Yang JC, Rosenberg SA. Science. 2014 May 9;344(6184):641-5. Cancer immunotherapy based on mutation-specific CD4+ T cells in a patient with epithelial cancer.

van Olffen RW, Koning N, van Gisbergen KP, Wensveen FM, Hoek RM, Boon L, Hamann J, van Lier RA, Nolte MA. J Immunol. 2009 Jun 15;182(12):7490-500. GITR triggering induces expansion of both effector and regulatory CD4+ T cells in vivo.

Vu MD, Xiao X, Gao W, Degauque N, Chen M, Kroemer A, Killeen N, Ishii N, Li XC. Blood. 2007. OX40 costimulation turns off Foxp3+ Tregs.

Watanabe N, Gavrieli M, Sedy JR, Yang J, Fallarino F, Loftin SK, Hurchla MA, Zimmerman N, Sim J, Zang X, Murphy TL, Russell JH, Allison JP, Murphy KM.Nat Immunol. 2003 Jul;4(7):670-9. BTLA is a lymphocyte inhibitory receptor with similarities to CTLA-4 and PD-1.

Xu X, Xu L, Ding S, Wu M, Tang Z, Fu W, Ni Q. Chin Med Sci J. 1995 Sep;10(3):185-7. Treatment of 23 patients with advanced gastric cancer by intravenously transfer of autologous tumor-infiltrating lymphocytes combined with rIL-2.

## Claims

1. A method for obtaining a cell population enriched for tumour reactive T-cells wherein T-cells expressing CD150 are selected and optionally expanded wherein the T-cells expressing CD150 are T-cells selected from cells originating from a patient and the cell population is selected from a population of tumour infiltrating lymphocytes (TILs) from a tumour biopsy, lymph node or ascites.

2. A method according to claim 1 wherein selecting the T-cells expressing CD150 comprises one or more of (i) flow cytometry, (ii) antibody panning, (iii) magnetic selection, (iv) biomarker targeted cell enrichment.

3. A method according to claim 1 wherein selecting the T-cells comprises contacting the cell population with an anti- CD150 antibody.

4. A method according to any preceding claim, wherein the T-cells expressing CD150/SLAM/SLAMF1 are expanded by way of one or both of the following options:
expansion with irradiated feeder cells to provide a T-cell activation signal and costimulation driven by antibodies or costimulatory receptors; or
expansion with immobilised or soluble reagents which provide a T-cell activation signal and costimulation signals driven through CD150.

5. A population of tumour infiltrating lymphocytes (TILs) obtained from a tumour biopsy, lymph node or ascites of a patient and enriched for tumour reactive T-cells autologous to the patient expressing CD150 for use in the treatment of cancer in said patient wherein at least 25% of the T-cells express CD150.

6. A population for use according to claim 5 wherein the cancer is selected from melanoma, ovarian or cervical cancer.

7. A population for use according to claim 5 or 6 wherein at least 30%, 40%, or 50% of the T-cells express SLAM/SLAMF1/CD150.

8. A population for use according to claim 5 or 6 wherein >25%, >30%, >35%, or >40% of the T-cells express SLAM/SLAMF1/CD150.

9. A method according to any of claims 1 to 4, or a population of cells for use according to any of claims 5 to 8, wherein the T-cells expressing CD150, comprise a CD4+ or a CD8+ cell, optionally wherein the T-cells expressing CD150 comprise a memory cell.

10. A method for assessing the tumour reactivity of a cell population which method comprises quantifying the proportion of T-cells expressing CD150 in the cell population wherein the cell population is i) TILs from a human patient and the T-cells expressing CD150 are quantified pre-REP and/or post-REP; or ii) a population of T-cells engineered to express an exogenous CAR and/or a TCR wherein said method is not a method of treatment or a diagnostic method performed on the animal or human body.

11. A method according to claim 10 wherein the proportion of T-cells expressing CD150 being at least 25% of the cell population indicates that the cell population is tumour reactive.

## Patentansprüche

1. Ein Verfahren zum Erhalten einer mit tumorreaktiven T-Zellen angereicherten Zellpopulation, wobei CD150 exprimierende T-Zellen ausgewählt und optional expandiert werden, wobei die CD150 exprimierenden T-Zellen T-Zellen sind, die aus Zellen ausgewählt werden, die von einem Patienten stammen, und die Zellpopulation aus einer Population tumorinfiltrierender Lymphozyten (TILs) aus einer Tumorbiopsie, einem Lymphknoten oder Aszites ausgewählt wird.

2. Verfahren gemäß Anspruch 1, wobei das Auswählen der CD150 exprimierenden T-Zellen eines oder mehrere von (i) Durchflusszytometrie, (ii) Antikörper-Panning, (iii) magnetischer Auswahl, (iv) Biomarker gerichteter Zellanreicherung beinhaltet.

3. Verfahren gemäß Anspruch 1, wobei das Auswählen der T-Zellen das In-Kontakt-Bringen der Zellpopulation mit einem Anti-CD150-Antikörper beinhaltet.

4. Verfahren gemäß einem der vorhergehenden Ansprüche, wobei die CD150/SLAM/SLAMF1 exprimierenden T-Zellen durch eine oder beide der folgenden Optionen expandiert werden:
Expansion mit bestrahlten Feeder-Zellen, um ein T-Zell-Aktivierungssignal und
Costimulation bereitzustellen, die durch Antikörper oder costimulatorische Rezeptoren angetrieben werden; oder
Expansion mit immobilisierten oder löslichen Reagenzien, die ein T-Zell-Aktivierungssignal und Costimulationssignale bereitstellen, die durch CD150 angetrieben werden.

5. Eine Population von tumorinfiltrierenden Lymphozyten (TILs), erhalten aus einer Tumorbiopsie, einem Lymphknoten oder Aszites eines Patienten und angereichert mit tumorreaktiven, autologen T-Zellen des Patienten, die CD150 exprimieren, zur Verwendung bei der Behandlung von Krebs bei dem Patienten, wobei mindestens 25 % der T-Zellen CD150 exprimieren.

6. Population zur Verwendung gemäß Anspruch 5, wobei der Krebs aus Melanom, Eierstock- oder Gebärmutterhalskrebs ausgewählt ist.

7. Population zur Verwendung gemäß Anspruch 5 oder 6, wobei mindestens 30 %, 40 % oder 50 % der T-Zellen SLAM/SLAMF1/CD150 exprimieren.

8. Population zur Verwendung gemäß Anspruch 5 oder 6, wobei > 25 %, > 30 %, > 35 % oder > 40 % der T-Zellen SLAM/SLAMF1/CD150 exprimieren.

9. Verfahren gemäß einem der Ansprüche 1 bis 4 oder eine Zellpopulation zur Verwendung gemäß einem der Ansprüche 5 bis 8, wobei die CD150 exprimierenden T-Zellen eine CD4+- oder eine CD8+-Zelle beinhalten, wobei die CD150 exprimierenden T-Zellen optional eine Gedächtniszelle beinhalten.

10. Ein Verfahren zur Bewertung der Tumorreaktivität einer Zellpopulation, wobei das Verfahren die Quantifizierung des Anteils an CD150 exprimierenden T-Zellen in der Zellpopulation beinhaltet, wobei die Zellpopulation Folgendes ist: i) TILs von einem menschlichen Patienten und die CD150 exprimierenden T-Zellen vor dem REP und/oder nach dem REP quantifiziert werden; oder ii) eine Population von T-Zellen, die so manipuliert wurden, dass sie ein exogenes CAR und/oder einen TCR exprimieren, wobei das Verfahren kein Behandlungsverfahren oder kein diagnostisches Verfahren ist, das am tierischen oder menschlichen Körper durchgeführt wird.

11. Ein Verfahren gemäß Anspruch 10, wobei die Tatsache, dass der Anteil an CD150-exprimierenden T-Zellen mindestens 25 % der Zellpopulation beträgt, anzeigt, dass die Zellpopulation auf einen Tumor anspricht.

## Revendications

1. Un procédé pour obtenir une population cellulaire enrichie en cellules T réactives à une tumeur, où des cellules T exprimant CD150 sont sélectionnées et facultativement expansées où les cellules T exprimant CD150 sont des cellules T sélectionnées à partir de cellules émanant d'un patient et la population cellulaire est sélectionnée à partir d'une population de lymphocytes infiltrant les tumeurs (TIL) provenant d'une biopsie d'une tumeur, d'un noeud lymphatique ou d'une ascite.

2. Un procédé selon la revendication 1 où la sélection des cellules T exprimant CD150 comprend un ou plusieurs éléments parmi (i) la cytométrie en flux, (ii) le panning avec des anticorps, (iii) la sélection magnétique, (iv) l'enrichissement cellulaire ciblé par biomarqueur.

3. Un procédé selon la revendication 1 où la sélection des cellules T comprend la mise en contact de la population cellulaire avec un anticorps anti-CD150.

4. Un procédé selon n'importe quelle revendication précédente, où les cellules T exprimant CD150/SLAM/SLAMF1 sont expansées au moyen de l'une ou des deux options suivantes :
une expansion avec des cellules nourricières irradiées afin de fournir un signal d'activation des cellules T et une costimulation induite par des anticorps ou des récepteurs de costimulation ; ou
une expansion avec des réactifs immobilisés ou solubles qui fournissent un signal d'activation des cellules T et des signaux de costimulation induits par le biais de CD150.

5. Une population de lymphocytes infiltrant les tumeurs (TIL) obtenue à partir d'une biopsie d'une tumeur, d'un noeud lymphatique ou d'une ascite d'un patient et enrichie en cellules T réactives à la tumeur autologues au patient exprimant CD150 pour une utilisation dans le traitement d'un cancer chez ledit patient où au moins 25 % des cellules T expriment CD150.

6. Une population pour une utilisation selon la revendication 5 où le cancer est sélectionné parmi un mélanome, un cancer de l'ovaire ou du col de l'utérus.

7. Une population pour une utilisation selon la revendication 5 ou la revendication 6 où au moins 30 %, 40 %, ou 50 % des cellules T expriment SLAM/SLAMF1/CD150.

8. Une population pour une utilisation selon la revendication 5 ou la revendication 6 où > 25 %, > 30 %, > 35 %, ou > 40 % des cellules T expriment SLAM/SLAMF1/CD150.

9. Un procédé selon n'importe lesquelles des revendications 1 à 4, ou une population de cellules pour une utilisation selon n'importe lesquelles des revendications 5 à 8, où les cellules T exprimant CD150 comprennent une cellule CD4+ ou une cellule CD8+, facultativement où les cellules T exprimant CD150 comprennent une cellule à mémoire.

10. Un procédé pour évaluer la réactivité à une tumeur d'une population cellulaire, lequel procédé comprend la quantification de la proportion de cellules T exprimant CD150 dans la population cellulaire, où la population cellulaire est i) des TIL provenant d'un patient humain et les cellules T exprimant CD150 sont quantifiées avant le REP et/ou après le REP ; ou ii) une population de cellules T modifiées pour exprimer un CAR exogène et/ou un TCR, ledit procédé n'étant pas un procédé de traitement ou un procédé diagnostique réalisé sur le corps animal ou humain.

11. Un procédé selon la revendication 10 où le fait que la proportion de cellules T exprimant CD150 est d'au moins 25 % de la population cellulaire indique que la population cellulaire est réactive à la tumeur.
